# EUROPEAN PATENT SPECIFICATION

(11) **EP 2 613 833 B1**
(45) Date of publication and mention of the grant of the patent: **10.01.2018**
(21) Application number: 11764304.9
(22) Date of filing: 29.08.2011
(51) Int. Cl.: A61M 16/00, A61M 16/01, A61M 16/12, A61M 16/20

(54) **LUNG VENTILATOR AND/OR ANAESTHESIA MACHINE**
LUNGENBEATMUNGSGERÄT UND/ODER NARKOSEGERÄT
RESPIRATEUR ET/OU MACHINE POUR ANESTHÉSIE

(30) Priority: 08.09.2010 US 381056 P; 07.09.2010 EP 10175668
(43) Date of publication of application: 17.07.2013
(73) Proprietor: iAsset ag, 9470 Buchs SG (CH)
(72) Inventor: FRIBERG, Harri, CH-9493 Mauren (LI); DAESCHER, Jakob, CH-7306 Flaesch (CH)
(74) Representative: Patentbüro Paul Rosenich AG
(86) International application number: PCT/IB2011/053772
(87) International publication number: WO 2012/032434

(56) References cited:
- WO-A1-98/09677
- WO-A1-99/13932
- WO-A2-2008/092021
- US-A1- 2007 175 473
- US-A1- 2009 241 960

## Description

The invention relates to a lung ventilator and/or anaesthesia machine with a blender for mixing a pressurised medical gas, e.g. oxygen, with air drawn in by a blower, comprising a gas line for the medical gas and an air line, which open into a common breathing gas line.

Known lung ventilators blend oxygen and air, whereby both gases are under high pressure (approximately 2 to 7 bars) and are supplied either via the hospital's high pressure pipeline system or from gas bottles. Both gases are mixed at a certain ratio (e.g. 1:1) and supplied to the patient during the breathing cycle. Mixing generally takes place in that the gases are fed into a blender tank (usually via on/off valves) arranged upstream of the ventilation mask or ventilation tube, and from there are supplied to the patient via a controlled ventilation valve.

There are also variants in which both gases - coming from two separate pressurised lines - are only mixed directly at the outlet of the lung ventilator and supplied to the patient via two controlled ventilation valves (proportional valves), one for the compressed air and one for the high-pressure oxygen. Both proportional valves synchronously dose the gas flow to the patient resulting in the required quantity of gas and the required gas blend.

However these methods only operate without fault if a high-pressure system of around the same pressure can be provided for both gases. A fundamental drawback is that compressed air is required in the first place. In addition the decompression of the compressed air produces cold. For this reason in the case of external moistening of the breathing air the patient tubes have to be heated as otherwise the moisture will condense.

In a new type of lung ventilator the air flow and/or air pressure is produced by a blower in the form of a fan wheel (also known as a blower or turbine). The advantage of this is that the air does not have to be available as compressed air, but can simply be drawn in from the surrounding atmosphere. In such systems, in order to add oxygen (or another gas or gas blend) from a high-pressure reservoir (hospital network or oxygen bottle), oxygen is blown via a valve into the intake area of the blower so that in addition to air the blower also transports oxygen to the ventilation valve before the ventilation mask.

However, this design is problematical. Although it solves the problem of mixing high-pressure oxygen to a relatively low-pressure air source, it has the following disadvantages:

The pure oxygen is conveyed via the relatively hot blowers resulting in a greatly increased risk of fire. The oxygen cannot be dosed precisely, which results in poor ventilation and/or waste of relatively expensive oxygen. If the patient does not inhale, an unnecessarily large amount of oxygen is supplied and wasted.

The prior art is explained in more detail below with the aid of a number of documents:
US 4 022 234 A discloses a ventilation system in which two different gases are mixed with each other at a certain ratio before being supplied to the breathing gas line. For this purpose a pressure chamber is provided into which the different gases are consecutively fed. As soon as the pressure in the chamber falls to a certain value gas A is initially introduced. If as a result of this the pressure increases to a further threshold value, gas B is then introduced until a predetermined final pressure is reached. As soon as this final pressure is reached the gas mixture is released from the pressure change and fed into a reservoir from which the breathing gas is taken.

A gas mixture of this type is associated with disadvantages as continuous mixing of gas is not possible. Also, pressure measurement can be erroneous so that no precise mixing of the gases in accordance with desired concentration values can take place. Furthermore, the system requires an additional reservoir to the pressure chamber which considerably increases the space requirements.

US 4 587 967 A discloses a variable flow piston lung ventilator in which oxygen is supplied from an oxygen tank (oxygen bottle). In the oxygen line there is a regulating valve (proportional valve) which produces an oxygen flow in proportion to a pulse width modulated signal. The oxygen flows into a blending chamber into which in another line air is fed from a piston pump. The breathing gas is supplied to the patient directly from the blending chamber, with a sensor being provided between the blending chamber and the patient which determines the oxygen content of the breathing air. All the sensors are connected to a control device, as is the piston pump.

The disadvantage of such as system is that measurement of the oxygen content can associated with errors and is strongly dependent on temperature, humidity, gas flow and suchlike. Also, a continuous gas flow cannot be provided by the piston system. The dosed quantity of breathing gas cannot therefore be finely and precisely adjusted.

US 5 383 449 A discloses a control system for mixing and intermittently supplying gas. This system uses to pressurised gas sources, oxygen and air. The supply lines of both gases open into a large pressurised container in which the gases are mixed. The breathing gas is taken from this pressurised container in the required quantity.

The particular disadvantage is the large pressurised container for storing the gas mixture which greatly increases the space requirement and the weight.

WO 2008/092021 A2 discloses a system with a blender for mixing a pressurised medical gas, e.g. oxygen, with air drawn in by a blower, comprising a gas line for the medical gas, and an air line, which open into a common breathing gas line. The opening of the gas line into the common gas line is downstream of the blower. In the gas line a regulating valve is provided for variable adjustment of the gas flow and in the common breathing gas line a regulating valve is provided for dosing the breathing gas. In the breathing gas line a flow sensor is provided for measuring the breathing gas flow. US 2009/0241960 A1 discloses a further dual high low pressure breathing system of such a kind.

WO 9809677 A1 discloses an oxygen mixing arrangement in a pressure support ventilator having a modular oxygen-providing assembly. The arrangement comprises a gas line for oxygen, and an air line, which open into a common breathing gas line. Air is drawn in by a blower arranged in the air line. The minimization of excess flow (i.e. venting) of oxygen is achieved by injecting the oxygen into the general airflow of the respirator apparatus at a point downstream from any pressure relief valve or other components capable of venting air and/or gas into the ambient atmosphere.

WO 9913932 A1 discloses a ventilator system which may be operated in an invasive and non-invasive mode. The system comprises a gas line for oxygen, and an air line, which open into a common breathing gas line. The gas line comprises an oxygen flow sensor measuring the flow of oxygen out of an oxygen flow valve. This measurement is used for closed loop control of oxygen flow valve. The air line comprises an air flow sensor measuring the flow of air out of a blower valve. This measurement is used for closed loop control of blower valve.

An aim of the invention is to create a new lung ventilator which on the one hand operates with known low-pressure blower technology and on the other hand with known high-pressure oxygen, but without the aforementioned fire risk, without wasting oxygen and with precise dosing of the mixture of air and oxygen or another medical gas, such as, for example narcotics, nitrogen, CO₂ etc. More particularly the objective of the invention is to eliminate the disadvantages occurring in the prior art and to provide a lung ventilator and/or anaesthesia machine with which the blending of a medical gas and air can be precisely controlled. In addition the quantity of breathing gas dosed to the patient should be optimally controllable. In particular the new system should guarantee that the oxygen concentration in the breathing gas is kept constant irrespective of influences from the breathing cycle. The invention should also ensure that the space requirement and weight of the lung ventilator are kept low and that safety is increased.

The aims are achieved with a lung ventilator and/or anaesthesia machine having the features as claimed in claim 1.

The measure allows a precisely regulable supply of a medical gas to the air and at the same time precise dosing of the quantity of breathing gas. In doing so mixing of a high-pressure medical gas with low-pressure air drawn in from the surrounding takes place. Even though in the following the term "oxygen" is sometimes used, the invention is applicable to every medical gas. In the invention only air flows through the blower as a result of which no medical gas can be lost in the direction of the air inlet. In this way the required gas composition can be set more precisely.

With appropriate control the oxygen supply can be clearly regulated as a function of measuring parameters. The measuring parameters relate either to internal system parameters, such as flow quantities in the lines, or patient-specific parameters such as the breathing cycle. In this way oxygen can not only be blended in precisely, but also efficiently saved if it is not needed. In the exhalation phase this regulating valve in the gas line can be throttled. A regulating/proportional valve on the inhalation side ensures optimum quantity dosing of the gas blend in the direction of the patient. The interaction of the two regulating valves delivers a precisely adjusted gas blend. The invention is characterised by its small structure as no tank is required. A high peak flow for the patient results as the blower supplies plenty of flow irrespective of which specified oxygen concentration is regulated.

It can involve home lung ventilators as well as lung ventilators and/or anaesthesia machines used on intensive care wards and in operating theatres. Further pressure and/or gas flow and/or volume sensors can influence the flow to the patient.

A flow sensor in the gas line to measure the gas flow and a flow sensor in the breathing gas line to measure the breathing gas flow are provided. The measurements of the individual flow sensors can be correlated with each other so that, for example, from the ratio of each of the flow quantities precise conclusions can be drawn with regard to the concentration of the medical gas in the air. The regulating valve in the gas line can now be operated as a function of this measuring value processing. Via a feedback loop an optimum valve setting corresponding to a particular gas concentration can be set.

In one embodiment a valve for reducing the gas pressure is provided in the gas line, with the regulating valve being arranged downstream thereof. This measure has the advantage that the pressure of the medical gas before the regulating valve is already sharply reduced in comparison with the high-pressure reservoir, which simplifies and makes regulation of the gas flow more precise.

In one embodiment the valve for reducing the gas pressure can be variably adjusted. This allows continuous control of the valve and any setting of the pressure in the gas line before the regulating valve. Throttling of this reduction valve is advantageous in the following phases: if there is no breath on the ventilation valve, e.g. during breathing pauses, and the regulating valve is throttled or completely closed, a higher pressure would build up before the throttled regulating valve. On reopening of the regulating valve the pressure equalisation causes an excessively large amount of oxygen to flow into the breathing gas line, which would result in an excessive concentration of oxygen in the breathing gas. However, if the reduction valve is throttled at the same time as the regulating valve and excess pressure and excess of oxygen can be prevented. Like the evaluation of the measuring parameters this can be done in real time.

In one embodiment a safety valve is provided in the gas line, preferably between the valve for reducing the gas pressure and the regulating valve for variable adjusting of the gas flow, as a result of which the safety of the patient and staff can be further increased.

In one embodiment the blower is arranged in the air line so that the medical gas cannot come into contact with parts of the blower that have become warm though being in operation. This is particularly relevant for oxygen as the risk of fire can thereby be considerably reduced.

In one embodiment a check valve is provided in the air line between the blower and the opening of the air line into the breathing gas line to prevent the return flow of gas in the direction of the blower. This reduces the risk of fire in the case of oxygen even further, as even with changed pressure ratios the return flow of oxygen in the direction of the blower can be prevented. It also prevents oxygen reaching the outside and being wasted.

In one embodiment a pressure sensor is provided in the breathing gas line on the outlet side. With this pressure sensor the pressure of the breathing gas can be monitored before it is supplied to the patient. This pressure sensor can also be used to detect the breathing cycle which in turn is a control parameter for the regulating valves.

In one embodiment the flow of medical gas into the breathing gas line is exclusively brought about by the, possibly reduced, inherent pressure of the gas entering the gas line. No active conveying devices for the medical gas are therefore necessary in the gas line. As high-pressure gas connected to the lung ventilator uses the inherent pressure to be transported in the direction of the breathing gas line.

In one embodiment the regulating valve and the flow meter of the lung ventilator and/or anaesthesia machine are connected to a control unit which controls the regulating valve as a function of the measuring values of the flow sensor in the gas line and the flow sensor in the breathing gas line. The flow quantities / the ratio of the flow quantities with regard to each other are used as a control parameter for the regulating valve in the gas line. The measuring data are constantly evaluated in real time, if necessary compared with calibration table and correlated with each other and then form the starting point for real-time control of the regulating valve. In the event of deviations from the specified concentration, or when setting a new specified concentration, the regulating valve can be automatically readjusted.

In one embodiment the control unit controls the valve for reducing the gas pressure and/or the regulating valve for adjusting the gas flow as a function of the breathing cycle. In this embodiment not only are the flow measurements used a control parameters, but opening and throttling of the valve(s) also takes place as a function of the breathing cycle. Control takes place so that oxygen is only supplied when it is actually needed, as a result of which oxygen can be saved.

The method for operating a lung ventilator and/or anaesthesia machine, in which a breathing gas is provided by mixing a pressurised medical gas with air conveyed by a blower, comprising a gas line and an air line which open into a common breathing air line, is characterised in that the supply of the medical gas into the common breathing gas line takes place downstream of the blower and in that the supply of the medical gas into the breathing gas line is controlled by a regulating valve, preferably a proportional valve, arranged in the gas line and in that the breathing gas is dosed with a regulating valve in the breathing gas line.

In one embodiment the flow of the medical gas into breathing gas line is exclusively caused by the, possibly reduced, inherent pressure of the gas entering the gas line.

In one embodiment the regulating valve in the gas line is controlled as a function of the measuring values of a flow sensor provided in the gas line and a flow sensor provided in the breathing gas line.

The high-pressure oxygen is preferably supplied via a reduction valve in the gas line to a flow measurer and from there via a first controllable proportional valve into a breathing gas line. The blower also supplies air, preferably via a check valve, to this breathing gas line. On the outlet side of the breathing gas line is a second flow measurer and a second proportional valve (the ventilation valve).

Both proportional valves are controlled in real time and by the patient's breathing rhythm. The control device measures and compares both flow measurements so that the correct ratio of oxygen is mixed into the total flow in accordance with the preset values.

In one embodiment the measuring values of the pressure flow sensor are evaluated in real time and correlated, and the regulating valve is controlled in real time as a function of this measurement processing. In this way slight deviations from specified values can be detected immediately and compensated for by appropriate control of the regulating valve.

In one embodiment the regulation valve in the gas line is controlled as a function of the breathing cycle.

In one embodiment the pressure of the medical gas is reduced in the inlet side of the gas line by a valve which is arranged upstream of the regulating valve.

In one embodiment the valve for reducing the gas pressure as a function of the breathing cycle is controlled so that the valve is throttled if on the outlet side there is no breath detected on the breathing gas line.

In one embodiment the breathing cycle is determined by a gas pressure sensor arranged on the outlet side of the breathing gas line. It is of course also conceivable for the breathing cycle to be determined in another manner, e.g. by means of elastic chest straps to measure the expansion of the chest cage, measuring the oxygen content between breathing and exhaled air etc.

In one embodiment the return flow of the medical gas into the air line is prevented by a check valve arranged between the blower and the opening of the air line into the breathing line.

The lung ventilator and/or anaesthesia machine can of course also have an output unit or display, a computer, a control program as well as internal sensors and, naturally, at least one ventilation connection for patients. Also described is a respiratory apparatus comprising:
a medical gas line;
an air line;
a common breathing gas line, said medical gas line and said air line both opening into said common breathing gas line;
a blower in said air line, said air line opening into said common breathing gas line downstream of said blower;
in said medical gas line, a first regulating valve configured to variably adjust medical gas flow;
in said common breathing gas line, a second regulating valve configured to dose breathing gas;
in said common breathing gas line, a first flow sensor configured to measure breathing gas flow; and,
in said medical gas line, a second flow sensor configured to measure medical gas flow.
Preferably said first regulating valve is a proportional valve; and,
said second regulating valve is a proportional valve.
Preferably, respiratory apparatus further comprises:
in said medical gas line, a variably-adjustable reduction valve configured to reduce medical gas pressure, said first regulating valve being disposed downstream of said variably-adjustable reduction valve.
Preferably, respiratory apparatus further comprises:
a safety valve in said medical gas line, said safety valve being disposed between said reduction valve and said first regulating valve.
Preferably, respiratory apparatus further comprises:
a control unit operatively connected to said first and second flow sensors and to said first regulating valve to control said first regulating valve as a function of values measured by said first and second flow sensors.
Preferably, said control unit controls said reduction valve.
Preferably, said control unit controls said first regulating valve as a function of the breathing cycle.
Preferably, respiratory apparatus further comprises:
in said air line, a check valve configured to prevent return flow of gas towards said blower.
Preferably, said check valve is disposed downstream of said blower.
Preferably, respiratory apparatus further comprises:
a gas pressure sensor disposed in said breathing gas line.
Preferably, respiratory apparatus further comprises:
a control unit operatively connected to said first and second flow sensors and to said first regulating valve to control said first regulating valve as a function of values measured by said first and second flow sensors.
A method of operating a respiratory apparatus comprising steps of:
conveying air in an air line with a blower;
supplying pressurized medical gas in a medical gas line;
mixing the conveyed air and the pressurized medical gas in a breathing gas line;
controlling supply of the pressurized medical gas into the breathing gas line with a first regulating valve; and,
dosing breathing gas from the breathing gas line with a second regulating valve in the breathing gas line.
Preferably, method of operating a respiratory apparatus further comprises the step of:
controlling the first regulating valve as a function of the values measured by a first flow sensor in the breathing gas line, and as a function of the values measured by a second flow sensor in the medical gas line.
Preferably, method of operating a respiratory apparatus further comprises the step of:
evaluating and correlating measured values of the first and second flow sensors; and,
controlling the first regulating valve in real time as a function of the evaluating and correlating of measured values.
Preferably, method of operating a respiratory apparatus further comprises the step of:
controlling the first regulating valve as a function of the breathing cycle.
Preferably, method of operating a respiratory apparatus further comprises the step of:
determining the breathing cycle with a gas pressure sensor in the outlet side of the breathing gas line.
Preferably, method of operating a respiratory apparatus further comprises the step of:
reducing medical gas pressure with a reducing valve disposed upstream of the first regulating valve.
Preferably, method of operating a respiratory apparatus further comprises the step of:
controlling the reducing valve as a function of the breathing cycle so as to throttle the reducing valve when absence of breath is detected in the breathing gas line.
Preferably, method of operating a respiratory apparatus further comprises the step of:
preventing return flow of medical gas into the air line by a check valve arranged between the blower and breathing gas line.
Also described is a respiratory apparatus comprising:
a medical gas line;
an air line;
a common breathing gas line, said medical gas line and said air line both opening into said common breathing gas line;
a blower in said air line, said air line opening into said common breathing gas line downstream of said blower;
in said medical gas line, a first regulating valve configured to variably adjust medical gas flow;
in said common breathing gas line, a second regulating valve configured to dose breathing gas;
in said common breathing gas line, a first flow sensor configured to measure breathing gas flow;
in said medical gas line, a second flow sensor configured to measure medical gas flow;
in said medical gas line, a variably-adjustable reduction valve configured to reduce medical gas pressure, said first regulating valve being disposed downstream of said variably-adjustable reduction valve;
a safety valve in said medical gas line, said safety valve being disposed between said reduction valve and said first regulating valve;
in said air line, a check valve configured to prevent return flow of gas towards said blower, said check valve being disposed downstream of said blower;
a gas pressure sensor disposed in said breathing gas line;
a control unit operatively connected to said first and second flow sensors and to said first regulating valve to control said first regulating valve as a function of values measured by said first and second flow sensors, said control unit being operatively connected to control said reduction valve, and said control unit being operatively connected to control said first regulating valve as a function of the breathing cycle.

Further embodiments are set out in the figures and in the dependent patent claims. Reference in this specification to "one embodiment" or "an embodiment" means that a particular feature, structure, or characteristic described in connection with the embodiment is included in at least one embodiment of the disclosure. The appearances of the phrase "in one embodiment" in various places within this specification are not necessarily all referring to the same embodiment, nor are separate or alternative embodiments mutually exclusive of other embodiments. Moreover, various features are described which may be exhibited by some embodiments and not by others. Similarly, various requirements are described which may be requirements for some embodiments but not other embodiments. The reference symbol list forms part of the disclosure. On the basis of the figures the invention will be explained in more detail below using symbols and examples.

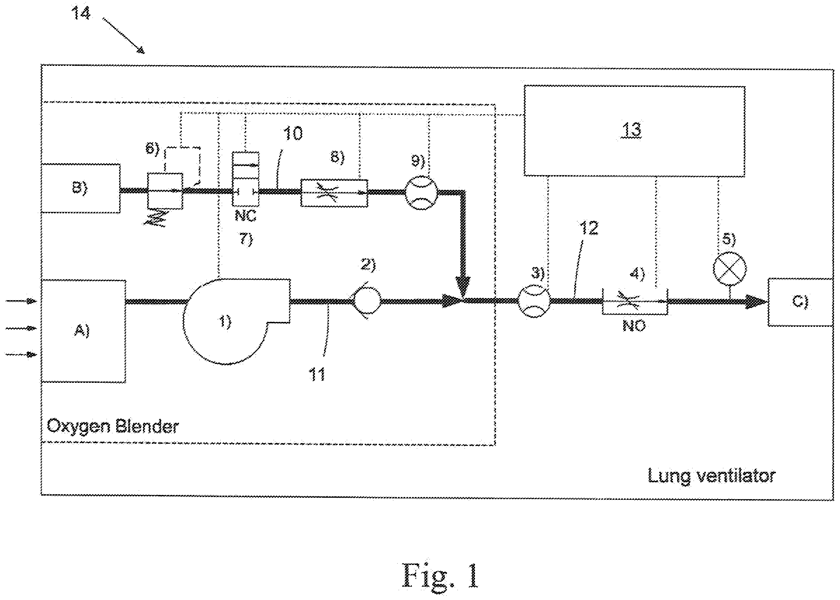

Fig.1 shows the block diagram of a lung ventilator in accordance with the invention.

Figure 1 shows a lung ventilator 14 in accordance with the invention, e.g. a (mobile) home lung ventilator or a lung ventilator or anaesthesia machine used on intensive care wards or in operation theatres. Functionally the lung ventilator 14 is divided into two areas, a blender, in which a medical gas, e.g. oxygen, nitrogen monoxide, various other anaesthetic, gaseous analgesics etc. is mixed with air, and a ventilations section via which the resulting gas mixture is supplied to the patient as breathing gas at an appropriate pressure and flow rate (quantity dosage). The lung ventilator 14 thus comprises a gas line 10 and an air line 11, which open into a common breathing air line 12.

A gas inlet B, for example in the form of a connection point for a high-pressure gas bottle or a connection for a high-pressure gas network opens into a gas line 10 into which the medical gas flows. On the inlet side the gas line 10 has a valve 6, usually a reduction valve with which the gas pressure is reduced downstream. Preferably this valve can be variably adjusted so the downstream gas pressure can be regulated at will. A regulating valve 8, a proportional valve, determines the flow of the medical gas in the direction of the common breathing gas line 12. A flow sensor 9 is integrated into the gas line 10 and in the shown example of embodiment is arranged downstream of the regulating valve 8. For safety purposes a safety valve 7 is provided between the reduction valve 6 and the regulating valve 8 which is activated in the event of failure or incorrect operation of the valve 6, i.e. closes if a pressure threshold is exceeded in the gas line 10. In a preferred embodiment the safety valve 7 is also controlled by the control unit 13. The reduction valve 6 can also be controlled by the control unit 13 or can be designed as a pneumatic pressure regulator.

The medical gas is exclusively conveyed in the gas line 10 by the inherent pressure of the gas stored in an external pressure reservoir and supplied via the gas line B. No active conveying devices are therefore necessary in the gas line 10.

An air inlet A, preferably fitted with a filter for cleaning the drawn in air, opens into an air line 11 in which a blower 1 provides active conveying of the drawn in air. Downstream of the blower 1 there is a check valve 2 in the air line 11 which prevents the return flow of the medical gas or gas mixture in the direction of air inlet A. The two lines 10 and 11 become a common breathing gas line 12 which already contains the gas mixture of required composition. The breathing gas line 12 has a flow sensor 3 and a regulating valve 4, preferably a proportional valve, and a pressure sensor 5. The regulating valve 4 is for dosing quantities of the gas mixture in accordance with the patient-specific, medical requirements.

All regulable valves and sensors in the lines as well as the blower are connected in terms of controlling and signalling to a control unit 13. The control/signalling leads are shown by a dotted and dashed line in fig. 1. An essential feature of one form of embodiment of the invention is that the regulating valve 8 in the gas line 10 is controlled by the control unit 13 in such a way that the gas mixture conveyed in the breathing gas line 12 is of the required composition. For this purpose the control unit 13 constantly, preferably continuously, reads out measurements recorded by the flow sensors 3 and 9, whereby a regulating parameter is determined from these measured values and used for regulating the regulating valve 8. After preparation of the measuring values with the aid of calibration data and/or taking into account other parameters such as temperature, pressure, humidity etc., the ratio of the flow quantity in the gas line 10 to the flow quantity in the gas line 12 plays an essential role. This ratio determines the proportion of the medical gas in the gas mixture. (In the case of oxygen it must of course be taken into account that air already contains a natural proportion of oxygen). If the results of the measurement evaluation deviate from a specified value or a specified composition of the breathing air the regulating valve 8 can be slightly readjusted resulting in a feedback loop.

In a particularly preferred form of embodiment the measuring values of the flow sensor are continuously evaluated in real time and provided as a regulating parameter. The valves are also controlled in accordance with this constantly changing regulating parameter so that the valves can also be adapted to the breathing cycle. For example if there is no breath detected at outlet C of the breathing gas line, the regulating valve 8 can be throttled in order not to supply oxygen unnecessarily. Equally the regulating valve 4 in the breathing gas line can be throttled in these phases. In order to avoid excessive pressure in the gas line 10 before the regulating valve 8 during throttling, the valve 6 for reducing the gas pressure is preferably also regulable. Depending on the throttling of the regulating valve 8, valve 6 is throttled accordingly so that the pressure between the two valves 6, 8 does not increase. Otherwise on subsequent opening of the regulating valve 8, due to the excess pressure an excessive amount of oxygen would be supplied to the air flow, which would result in distortion of the required gas composition in the breathing gas line.

In a preferred variant the blower 1 is also controlled as a function of the breathing cycle.

In order to record the breathing cycle a pressure sensor 5 is used on the output side in the breathing gas line 12. Valves 6, 8 and/or 4 are thus controlled in real time as a function of the measuring values of the pressure sensor 5. The breathing cycle can of course be recorded with any other method. At the breathing gas outlet C of the lung ventilator 14 a ventilation mask is usually connected by means of a flexible line.

### Reference symbol list

- 1 -: Blower
- 2 -: Check valve
- 3 -: Flow sensor
- 4 -: Regulating valve
- 5 -: Pressure sensor
- 6 -: Valve
- 7 -: Safety valve
- 8 -: Regulating valve
- 9 -: Flow sensor
- 10 -: Gas line
- 11 -: Air line
- 12 -: Breathing gas line
- 13 -: Control unit
- 14 -: Lung ventilator
- A -: Air inlet
- B -: Gas inlet
- C -: Breathing gas outlet

## Claims

1. Lung ventilator (14) and/or anaesthesia machine with a blender for mixing a pressurised medical gas, e.g. oxygen, with air drawn in by a blower (1), comprising a blower (1), a gas line (10) for the medical gas, and an air line (11), which open into a common breathing gas line (12), whereby the opening of the gas line (10) into the common gas line (12) is downstream of the blower (1) and in the gas line (10) a regulating valve (8), preferably a proportional valve, is provided for variable adjustment of the gas flow and in the common breathing gas line (12) a regulating valve (4), preferably a proportional valve, is provided for dosing the breathing gas, and whereby in the breathing gas line (12) a flow sensor (3) is provided for measuring the breathing gas flow, **characterised in that** in the gas line (10) a flow sensor (9) is provided for measuring the gas flow.

2. Lung ventilator and/or anaesthesia machine in accordance with claim 1, **characterised in that** at the inlet side a valve (6) is provided in the gas line (10) for reducing the gas pressure, whereby the regulating valve (8) is arranged downstream thereof, **whereby** the valve (6) for reducing the gas pressure can preferably be variably adjusted.

3. Ventilator and/or anaesthesia machine in accordance with any one of the preceding claims, **characterised in that** a safety valve (7) is provided in the gas line (11), preferably between the valve (6) for reducing the gas pressure and the regulating valve (8) for variable adjustment of the gas flow.

4. Lung ventilator and/or anaesthesia machine in accordance with any one of the preceding claims, **characterised in that** lung ventilator and/or anaesthesia machine comprises:
in said medical gas line (10), a variably-adjustable reduction valve (6) configured to reduce medical gas pressure, said regulating valve (8) in the gas line (10) being disposed downstream of said variably-adjustable reduction valve (6);
a safety valve (7) in said medical gas line (10), said safety valve (7) being disposed between said reduction valve (6) and said regulating valve (8);
in said air line (11), a check valve (2) configured to prevent return flow of gas towards said blower (1), said check valve (2) being disposed downstream of said blower (1);
a gas pressure sensor (5) disposed in said breathing gas line (12);
a control unit (13) operatively connected to said flow sensors (3, 9) and to said regulating valve (8) in the gas line (10) to control said regulating valve (8) in the gas line (10) as a function of values measured by said flow sensors (3, 9), said control unit (13) being operatively connected to control said reduction valve (6), and said control unit (13) being operatively connected to control said regulating valve (8) as a function of the breathing cycle.

5. Lung ventilator and/or anaesthesia machine in accordance with any one of the preceding claims, **characterised in that** a check valve (2) is provided in the air line (11) between the blower (1) and the opening of the air line (11) into the breathing gas line (12) which prevents the return flow of gas in the direction of the blower (1).

6. Lung ventilator and/or anaesthesia machine in accordance with any one of the preceding claims **characterised in that** a gas pressure sensor (5) is provided in the breathing gas line (12) at the outlet side.

7. Lung ventilator and/or anaesthesia machine in accordance with any one of the preceding claims, **characterised in that** the flow of the medical gas into the breathing gas line (12) is exclusively brought about by the, possibly reduced, inherent pressure of the gas entering the gas line (10).

8. Lung ventilator and/or anaesthesia machine in accordance with any one of the preceding claims, **characterised in that** the regulating valve (8) and the flow sensors (3, 9) of the lung ventilator and/or anaesthesia machine (14) are connected to a control unit (13) which controls the regulating valve (8) as a function of the measuring values of the flow sensor (9) in the gas line (10) and the flow sensor (3) in the breathing gas line (12).

9. Lung ventilator and/or anaesthesia machine in accordance with any one of the preceding claims, **characterised in that** the control unit (13) controls the valve (6) for reducing the gas pressure and/or the regulating valve (8) for the variable adjustment of the gas flow as a function of the breathing cycle.

## Patentansprüche

1. Beatmungsgerät (14) und/oder Anästhesiegerät mit einer Mischvorrichtung zum Mischen eines unter Druck stehenden medizinischen Gases, z. B. Sauerstoff, mit durch ein Gebläse (1) angesaugter Luft, umfassend ein Gebläse (1),
eine Gasleitung (10) für das medizinische Gas, und eine Luftleitung (11), welche in einer gemeinsamen Atemgasleitung (12) münden, wobei sich die Einmündung der Gasleitung (10) in die gemeinsame Gasleitung (12) stromabwärts des Gebläses (1) befindet und in der Gasleitung (10) ein Regelventil (8), vorzugsweise ein Proportionalventil, zur variablen Einstellung der Gasströmung vorgesehen ist, und in der gemeinsamen Atemgasleitung (12) ein Regelventil (4), vorzugsweise ein Proportionalventil, zur Dosierung des Atemgases vorgesehen ist, und wobei in der Atemgasleitung (12) ein Durchflusssensor (3) zur Messung der Atemgasströmung vorgesehen ist, **dadurch gekennzeichnet, dass** in der Gasleitung (10) ein Strömungssensor (9) zur Messung der Gasströmung vorgesehen ist.

2. Beatmungsgerät und/oder Anästhesiegerät nach Anspruch 1, **dadurch gekennzeichnet, dass** eingangsseitig ein Ventil (6) in der Gasleitung (10) zur Reduktion des Gasdrucks vorgesehen ist, wobei das Regelventil (8) stromabwärts davon angeordnet ist, wobei das Ventil (6) zur Reduktion des Gasdrucks vorzugsweise variabel einstellbar ist.

3. Beatmungsgerät und/oder Anästhesiegerät nach einem der vorherigen Ansprüche, **dadurch gekennzeichnet, dass** ein Sicherheitsventil (7) in der Gasleitung (11) vorgesehen ist, vorzugsweise zwischen dem Ventil (6) zur Reduktion des Gasdrucks und dem Regelventil (8) zur variablen Einstellung der Gasströmung.

4. Beatmungsgerät und/oder Anästhesiegerät nach einem der vorherigen Ansprüche, **dadurch gekennzeichnet, dass** das Beatmungsgerät und/oder Anästhesiegerät Folgendes umfasst:
in der medizinischen Gasleitung (10), ein variabel einstellbares Reduktionsventil (6), das konfiguriert ist, um den Druck des medizinischen Gases zu reduzieren, wobei das Regelventil (8) in der Gasleitung (10) stromabwärts des variabel einstellbaren Reduktionsventils (6) angeordnet ist;
ein Sicherheitsventil (7) in der medizinischen Gasleitung (10), wobei das Sicherheitsventil (7) zwischen dem Reduktionsventil (6) und dem Regelventil (8) angeordnet ist;
in der Luftleitung (11) ein Rückschlagventil (2), das konfiguriert ist, um eine Rückströmung von Gas zu dem Gebläse (1) hin zu verhindern, wobei das Rückschlagventil (2) stromabwärts des Gebläses (1) angeordnet ist;
einen Gasdrucksensor (5), der in der Atemgasleitung (12) angeordnet ist;
eine Steuereinheit (13), die mit den Durchflusssensoren (3, 9) und mit dem Regelventil (8) in der Gasleitung (10) wirkverbunden ist, um das Regelventil (8) in der Gasleitung (10) in Abhängigkeit von Werten, die von den Durchflusssensoren (3, 9) gemessen werden, zu steuern, wobei die Steuereinheit (13) wirkverbunden ist, um das Reduktionsventil (6) zu steuern, und wobei die Steuereinheit (13) wirkverbunden ist, um das Regelventil (8) in Abhängigkeit des Atemzyklus zu steuern.

5. Beatmungsgerät und/oder Anästhesiegerät nach einem der vorherigen Ansprüche, **dadurch gekennzeichnet, dass** ein Rückschlagventil (2) in der Luftleitung (11) zwischen dem Gebläse (1) und der Einmündung der Luftleitung (11) in die Atemgasleitung (12) vorgesehen ist, welches die Rückströmung von Gas in die Richtung des Gebläses (1) verhindert.

6. Beatmungsgerät und/oder Anästhesiegerät nach einem der vorherigen Ansprüche, **dadurch gekennzeichnet, dass** ein Gasdrucksensor (5) in der Atemgasleitung (12) ausgangsseitig vorgesehen ist.

7. Beatmungsgerät und/oder Anästhesiegerät nach einem der vorherigen Ansprüche, **dadurch gekennzeichnet, dass** die Strömung des medizinischen Gases in die Atemgasleitung (12) ausschließlich durch den, gegebenenfalls reduzierten, Eigendruck des in die Gasleitung (10) eintretenden Gases bewirkt wird.

8. Beatmungsgerät und/oder Anästhesiegerät nach einem der vorherigen Ansprüche, **dadurch gekennzeichnet, dass** das Regelventil (8) und die Durchflusssensoren (3, 9) des Beatmungsgeräts und/oder Anästhesiegeräts (14) mit einer Steuereinheit (13) verbunden sind, welche das Regelventil (8) in Abhängigkeit der Messwerte des Strömungssensors (9) in der Gasleitung (10) und des Durchflusssensors (3) in der Atemgasleitung (12) steuert.

9. Beatmungsgerät und/oder Anästhesiegerät nach einem der vorherigen Ansprüche, **dadurch gekennzeichnet, dass** die Steuereinheit (13) das Ventil (6) zur Reduktion des Gasdrucks und/oder das Regelventil (8) zur variablen Einstellung der Gasströmung in Abhängigkeit des Atemzyklus steuert.

## Revendications

1. Ventilateur pulmonaire (14) et/ou appareil d'anesthésie avec un mélangeur permettant de mélanger un gaz médical comprimé, par exemple de l'oxygène, avec de l'air introduit par un souffleur (1), comprenant un souffleur (1), une ligne de gaz (10) pour le gaz médical, et une ligne d'air (11), lesquelles débouchent sur une ligne de gaz respiratoire commune (12), où l'ouverture de la ligne de gaz (10) vers la ligne de gaz commune (12) se trouve en aval du souffleur (1), et où une soupape de régulation (8), de préférence une soupape proportionnelle, est prévue dans la ligne de gaz (10) pour un réglage variable du flux de gaz, et où une soupape de régulation (4), de préférence une soupape proportionnelle, est prévue dans la ligne de gaz respiratoire commune (12) pour le dosage du gaz respiratoire, et où un capteur de flux (3) est prévu dans la ligne de gaz respiratoire (12) pour la mesure du flux de gaz respiratoire, **caractérisé en ce qu'**un capteur de flux (9) est prévu dans la ligne de gaz (10) pour la mesure du flux de gaz.

2. Ventilateur pulmonaire et/ou appareil d'anesthésie selon la revendication 1, **caractérisé en ce que** du côté entrée, il est prévu une soupape (6) dans la ligne de gaz (10) pour réduire la pression du gaz, la soupape de régulation (8) étant disposée en aval de celle-ci, la soupape (6) destinée à réduire la pression du gaz pouvant être de préférence réglée de façon variable.

3. Ventilateur et/ou appareil d'anesthésie selon l'une quelconque des revendications précédentes, **caractérisé en ce qu'**une soupape de sécurité (7) est prévue dans la ligne de gaz (11), de préférence entre la soupape (6) destinée à réduire la pression du gaz et la soupape de régulation (8) pour le réglage variable du flux de gaz.

4. Ventilateur pulmonaire et/ou appareil d'anesthésie selon l'une quelconque des revendications précédentes, **caractérisé en ce que** le ventilateur pulmonaire et/ou l'appareil d'anesthésie comprend :
dans ladite ligne de gaz médical (10), une soupape de réduction réglable de façon variable (6), configurée pour réduire la pression du gaz médical, ladite soupape de régulation (8) dans la ligne de gaz (10) étant disposée en aval de ladite soupape de réduction réglable de façon variable (6) ;
une soupape de sécurité (7) dans ladite ligne de gaz médical (10), ladite soupape de sécurité (7) étant disposée entre ladite soupape de réduction (6) et ladite soupape de régulation (8) ;
dans ladite ligne d'air (11), une clapet antiretour (2) configuré pour empêcher le gaz de revenir vers ledit souffleur (1), ledit clapet antiretour (2) étant disposé en aval dudit souffleur (1) ;
un capteur de pression de gaz (5) disposé dans ladite ligne de gaz respiratoire (12) ;
une unité de commande (13) fonctionnellement reliée auxdits capteurs de flux (3, 9) et à ladite soupape de régulation (8) dans la ligne de gaz (10) pour commander ladite soupape de régulation (8) dans la ligne de gaz (10) en tant que fonction de valeurs mesurées par lesdits capteurs de flux (3, 9), ladite unité de commande (13) étant fonctionnellement reliée pour commander ladite soupape de réduction (6), et ladite unité de commande (13) étant fonctionnellement reliée pour commander ladite soupape de régulation (8) en tant que fonction du cycle respiratoire.

5. Ventilateur pulmonaire et/ou appareil d'anesthésie selon l'une quelconque des revendications précédentes, **caractérisé en ce qu'**un clapet antiretour (2) est prévu dans la ligne d'air (11) entre le souffleur (1) et l'ouverture de la ligne d'air (11) vers la ligne de gaz respiratoire (12) empêchant le gaz de revenir dans la direction du souffleur (1).

6. Ventilateur pulmonaire et/ou appareil d'anesthésie selon l'une quelconque des revendications précédentes, **caractérisé en ce qu'**un capteur de pression de gaz (5) est prévu dans la ligne de gaz respiratoire (12) du côté sortie.

7. Ventilateur pulmonaire et/ou appareil d'anesthésie selon l'une quelconque des revendications précédentes, **caractérisé en ce que** le flux du gaz médical dans la ligne de gaz respiratoire (12) est généré exclusivement par la pression inhérente du gaz entrant dans la ligne de gaz (10), éventuellement réduite.

8. Ventilateur pulmonaire et/ou appareil d'anesthésie selon l'une quelconque des revendications précédentes, **caractérisé en ce que** la soupape de régulation (8) et les capteurs de flux (3, 9) du ventilateur pulmonaire et/ou de l'appareil d'anesthésie sont reliés à une unité de commande (13) commandant la soupape de régulation (8) en tant que fonction des valeurs de mesure du capteur de flux (9) dans la ligne de gaz (10) et du capteur de flux (3) dans la ligne de gaz respiratoire (12).

9. Ventilateur pulmonaire et/ou appareil d'anesthésie selon l'une quelconque des revendications précédentes, **caractérisé en ce que** l'unité de commande (13) commande la soupape (6) pour réduire la pression du gaz et/ou la soupape de régulation (8) pour le réglage variable du flux de gaz en tant que fonction du cycle respiratoire.
